# EUROPEAN PATENT SPECIFICATION

(11) **EP 1 484 054 B1**
(45) Date of publication and mention of the grant of the patent: **29.08.2012**
(21) Application number: 03705377.4
(22) Date of filing: 21.02.2003
(51) Int. Cl.: A61K 9/08, A61K 9/14, A61K 31/573, A61K 47/06, A61K 47/32, A61K 47/36, A61P 27/02, A61P 29/00, A61P 31/04, A61P 31/10, A61P 31/12, A61P 35/00, A61P 37/06

(54) **DRUG DELIVERY SYSTEM FOR THE SUBCONJUNCTIVAL ADMINISTRATION OF FINE GRAINS**
ARZNEIMITTELABGABESYSTEM ZUR SUBKONJUNKTIVALEN VERABREICHUNG VON FEINEN KÖRNERN
SYSTEME D'ADMINISTRATION DE MEDICAMENT DESTINE A ADMINISTRER DE FACON SUBCONJONCTIVALE DES GRAINS FINS

(30) Priority: 22.02.2002 JP 2002046355
(43) Date of publication of application: 08.12.2004
(73) Proprietor: SANTEN PHARMACEUTICAL CO., LTD., Higashiyodogawa-ku, Osaka-shi, Osaka 533-8651 (JP)
(72) Inventor: KUWANO, Mitsuaki, SANTEN PHARMACEUTICAL CO., LTD.,, Ikoma-shi, Nara 630-0101 (JP); YAMADA, Kazuhito, SANTEN PHARMACEUTICAL CO., LTD.,, Ikoma-shi, Nara 630-0101 (JP)
(74) Representative: TER MEER - STEINMEISTER & PARTNER GbR
(86) International application number: PCT/JP2003/001897
(87) International publication number: WO 2003/070219

(56) References cited:
- WO-A-96/34599
- JP-A- 8 176 016
- JP-A- 2000 247 871
- JP-A- 2002 326 962
- US-A- 3 960 150
- US-A- 5 185 152
- US-A- 5 922 340
- ZIMMER A K ET AL: "Evaluation of pilocarpine-loaded albumin particles as drug delivery systems for controlled delivery in the eye I. In vitro and in vivo characterisation" JOURNAL OF CONTROLLED RELEASE, ELSEVIER, AMSTERDAM, NL, vol. 32, no. 1, November 1994 (1994-11), pages 57-70, XP004037676 ISSN: 0168-3659
- YATAKA NAKANO: 'Ryokunaisho shujutsu to 5-fluorouracil(5-FU)' RINSHO GANKA vol. 43, no. 13, 1989, pages 1929 - 1933, XP002975381

## Description

### Technical Field

The present invention relates to a drug delivery system to posterior segments such as a retina, a choroid and an optic nerve.

### Background Art

Diseases of posterior segments such as a retina, a choroid and an optic nerve are often intractable, and a development of an effective treatment method is eagerly desired. Though ophthalmopathy is most generally treated by instillation of drugs, the drugs are hardly delivered to the posterior segments such as a retina, choroid and an optic nerve. Even if the drugs are delivered to the posterior segments, it is very difficult to sustain a drug concentration in those tissues.

In view of this, an intravenous injection, oral administration and a vitreous injection are attempted to administer the drugs for the diseases of the posterior segments. However, the intravenous injection and the oral administration can deliver only a very minute amount of drugs to the posterior segments which are target sites, and sometimes causes unexpected strong systemic actions (side effects) of the drugs.

In the case of the vitreous injection, since the drug is directly injected into eyes, the amount of the drug to be delivered to the posterior segments is larger than those of the intravenous injection and the oral administration. The delivery to the posterior segments by the vitreous injection is summarized in Journal of ocular pharmacology and therapeutics, (2001) 17/4, 393-401 as a review. However, the vitreous injection is a method of administration which requires skilled procedure and is accompanied by a considerable pain. Accordingly, burdens on patients are heavy, and it is very difficult to administer the drug plural times.

Unlike these methods of administration, a subconjunctival injection, of which procedure is relatively easy, hardly causes disorders of ophthalmic tissues and burdens on patients are light, compared with the vitreous injection. A delivery of a drug to the posterior segments after the subconjunctival injection was reported (Invest. Ophthalmol. Visual Sci. 18 (3) 250-255, 1979), but its half-life was remarkably short, and it was difficult to sustain a drug concentration in the posterior segment tissues for a long period. Accordingly, frequent administration is required in order to sustain the drug concentration in the tissues, but the frequent administration increases the burdens on patients.

Known methods of sustaining the drug concentration in eyes without doing the frequent administration are exemplified by a method of administrating a conjugate of a drug with a polymer intravenously (Invest. Ophthalmol. Visual Sci. 40 (1), 2690-2696, 1999), a method of injecting a microsphere containing a drug into a vitreous body (Japanese Laid-open Patent Publication No. 247871/2000).

Since it was difficult to sustain the concentration of the drug injected subconjunctivally into the tissues by the conventional techniques as mentioned above, it was desired to develop a sustained drug delivery system to the posterior segments by the subconjunctival injection.

### Disclosure of the Invention

Studying precisely, the present inventors found that subconjunctival administration of sustained release fine particles containing a drug is very useful as a sustaining drug delivery system to posterior segments.

The present invention relates to the drug delivery system to the posterior segments to be used in order to administer the fine particles containing the drug subconjunctivally. The present invention also relates to a subconjunctival injection which comprises the fine particles containing the drug and enables the drug to deliver to the posterior segments. The delivery of drug to the posterior is excellent and systemic side effects are hardly caused by administering the fine particles containing the drug compared with an intravenous injection and oral administration. Procedure is easy and burdens on patients are light compared with a vitreous injection. Further, a drug concentration in the target tissue can be sustained for a long period by using the fine particles containing the drug.

The materials used to form the fine particles in the present invention are biodegradable or biosoluble polymers, and specific examples thereof are biodegradable polymers such as poly(lactic acid), lactic acid-glycolic acid copolymers, lactic acid-caprolactone copolymers, polyanhydrides, poly (ortho ester), poly ε -caprolactone, polyacrylcyanoacrylates, polyhydroxyalkanoates, polyphosphoesters, polyamino acids and poly α-hydroxyacids; natural polymers such as gelatin, collagen, hyaluronic acid, dextran, starch, sodium alginate, agar, pullulan, albumin, carageenan, pectin, xanthan gum, gellan gum, casein, chitosan and fibrinogen; and synthetic polymers such as methacrylic acid copolymers, polyvinyl alcohol, hydroxypropylmethylcellulose, hydroxypropylmethylcellulose acetate, hydroxyethyl cellulose, carboxymethylcellulose, methyl cellulose, polyvinyl pyrrolidone, polyethylene glycol and poly N-alkylacrylamide.

Molecular weight of these polymeric substances is not particularly limited and can be appropriately selected depending on the kind of drug contained in the fine particles, an effective drug concentration for treatment, a release period of the drug or the like.

The particle diameter of the fine particles in the present invention is 50 nm to 150 µ m. It is difficult to produce fine particles having a particle diameter of 50 nm or less. The particle diameter of 150 µm or more is too large to use the fine particles in the form of injections. A more preferred particle diameter is 200 nm to 75 µm.

The drug delivery system of the present invention is used for treatment or prevention of diseases of a retina, a choroid membrane and an optic nerve. Specific examples of diseases are inflammation due to various causes, viral or bacterial infections, diseases due to angiogenesis of a retina-choroid, diseases due to ischemia of a retina and optic nerve disorders due to glaucoma. Further specific examples of diseases are uveitis, cytomegalovirus retinitis, age-related macular degeneration, diabetic retinopathy, proliferative vitreoretinopathy, retinal detachment, pigmentary retinal degeneration, central retinal vein occlusion and central retinal artery occlusion.

The drugs contained in the fine particles are not particularly limited, and drugs suited for object diseases can be selected. Specific examples of drugs are steroids or derivatives thereof such as betamethasone, dexamethasone, triamcinolone, prednisolone, fluorometholone, hydrocortisone and progesterone; anti-inflammatoriessuch as bromofenac and diclofenac; cytokine inhibitors such as TNF-α inhibitors, PDE-IV inhibitors and ICE inhibitors; immunosuppressors such as ciclosporin and tacrolimus; antivirals such as ganciclovir, aciclovir and interferon-β; antimicrovials such as ofloxacin, clarithromycin and erythromycin; carcinostatic agents such as fluorouracil, methotrexate and MMP inhibitors; angiogenesis inhibitors such as endostatin, VEGF inhibitors, antisense oligonucleotide, PKC inhibitors, adhesion factor inhibitors and vascular resting steroid; neural protectants-neural nutrition factors such as MK-801, timolol, creatine, taurine and BDNF; carbonate dehydratase inhibitors such as acetazolamide; and thrombolytic drugs such as urokinase. Preferred forms of the fine particles containing the drug are a matrix-type wherein the drug is dispersed uniformly in the fine particles and a capsule-type wherein the drug as a core is encapsulated with the fine particles.

An amount of the drug contained in the fine particles can be appropriately increased or reduced depending on the kind of drug, the effective drug concentration for treatment, the release period of the drug, symptoms of diseases or the like. A drug content is 0.01 to 95% by weight, preferably 0.1 to 20% by weight in the fine particles.

The fine particles can be produced by a grinding method using a mill, a phase separation method (a coacervation method), a spray drying method, a supercritical fluid method, an interfacial deposition method or an interfacial reaction method, which is known, and the method is not limited to them. More specific examples of methods are a submerged drying method, which is the interfacial deposition method (J. Control. Release, 2, 343-352, (1985)), an interfacial polymerization method, which is an interfacial reaction method (Int. J. Pharm., 28, 125-132 (1986)) and a self-emulsification solvent diffusion method (J. Control. Release, 25, 89-98 (1993)). An appropriate process for production can be selected among these processes for production considering the particle diameter of the fine particles, the kind, properties or a content of the contained drug or the like.

A practical production example of drug-containing fine particles will be illustrated later in which fine particles contain betamethasone, an anti-inflammatory, and the material of the fine particles is polylactic acid.

Effects of the present invention will be described later in detail in a section of "drug concentration in retina-choroid measurement tests". Administering the fine particles containing betamethasone subconjunctivally and measuring a drug concentration in a retina-choroid, it was found that the drug concentration in the retina-choroid is sustained.

The fine particles in the drug delivery system of the present invention are administered subconjunctivally. The subconjunctival administration can be carried out using an ordinary subconjunctival injection. The procedure of the subconjunctival injection is relatively easy, and the burdens on patients are light as described in the section of "Background Art".

Further, since the drug can be efficiently delivered to the posterior segments such as a retina, a choroid and an optic nerve by using the system of the present invention, a dosage of the drug can be reduced, and consequently side effects can also be reduced.

To administer subconjunctivally the fine particles to be used for the drug delivery system of the present invention, the dosage forms are injections. The injections can be prepared by widely-used formulation techniques of injections. For example, the preparations can be prepared by adding an additive to be usually used such as an osmotic pressure adjustor such as sodium chloride, a buffer such as sodium phosphate, a surfactant such as polysorbate 80 or a thickener such as methyl cellulose and the fine particles to distilled water for injections. When a high pressure syringe having no needle is used, the fine particles can be administered as they are without rendering them the injection.

A production example of the fine particles, an example of the preparation and results of drug concentration measurement tests and choroidal neovascularization inhibition tests are illustrated below.

### Best Mode for Carrying out the Invention

### 1. Process for producing drug-containing fine particles

A production example of fine particles which can be used for a drug delivery system of the present invention is illustrated below.

Betamethasone (0.025 g) and polylactic acid (0.25 g) having weight-average molecular weight of 20,000 were dissolved in benzyl alcohol (1.5 ml). The obtained solution was referred to as a drug/polymer solution. A 2.0% (w/v) aqueous polyvinyl alcohol solution (30 ml) was homogenized with a homogenizer (5,000 rpm), and the drug/polymer solution was added dropwise to the homogenized solution. The mixture was homogenized for five minutes after finishing dropping to prepare an O/W emulsion. Ultrapure water (300 ml) was stirred (300 rpm) with a stirrer, thereto the prepared O/W emulsion was added dropwise followed by stirring for one hour after finishing dropping. After stirring, the obtained suspension was centrifuged, and the resulting supernatant was removed. In order to wash the resulting precipitate, ultrapure water (30 ml) was added to the precipitate to disperse it, the dispersion was centrifuged again, and the resulting supernatant was removed. This operation was repeated one more time. The washed precipitate was sieved to give particles having particle diameters of 50 nm to 75 µm. The obtained particles were lyophilized to give betamethasone-containing microsphere.

### 2. Process for preparing preparation

Betamethasone-containing microsphere powder (442 mg) was dispersed in a solvent (4 ml of an aqueous solution containing 0.4% (w/v) of polysorbate 80 and 2.6% (w/v) of glycerin). The obtained dispersion was referred to as a betamethasone-containing microsphere injection.

### 3. Measurement of drug concentration in retina-choroid

Using the betamethasone-containing microsphere injection, a betamethasone concentration in a retina-choroid was measured according to the method below. As a control, using a betamethasone suspension, a concentration was measured in the same manner. A betamethasone concentration in the retina-choroid of a microsphere administration group was compared with that of a suspension administration group. The betamethasone suspension was prepared by suspending betamethasone in a solvent (an aqueous solution containing 0.4% (w/v) of polysorbate 80 and 2.6% (w/v) of -glycerin).
1) A 0.5% (w/v) oxybuprocaine hydrochloride ophthalmic solution was instilled into both eyes of Japanese white rabbits to anesthetize the eye surfaces.
2) The betamethasone-containing microsphere injection was subconjunctivally administered to an upper portion in an amount of 100 µl per eye with a syringe equipped with a 27 G needle. Since a betamethasone content in the microsphere was about 4.6% (w/v), a dosage of betamethasone was about500 µ g. A 1% (w/v) betamethasone suspension was subconjunctivally administered to an upper portion of a control group in an amount of 50 µl per eye with the syringe equipped with the 27 G needle.
3) The rabbits were killed on 2nd, 7th, 14th, 21st and 28th day after administration respectively. After enucleation of eyeball, the retina-choroids were recovered. Then betamethasone concentrations in the retina-choroids were measured with a high performance liquid chromatograph.

Results of changes in drug concentration with time are shown in Table 1. As apparent from Table 1, in the case of the betamethasone suspension, the betamethasone concentration in the retina-choroid was about 0.96 µg/g tissue after seven days, but it was a detection limit or lower after 14 days. To the contrary, in the case of the betamethasone-containing microsphere, the betamethasone concentration in the retina-choroid was about 0.09 µg/g tissue even after 28 days, and the drug concentration in the retina-choroid was sustained.

**Table 1: Betamethasone concentrations in retina-choroids (µ g/g tissue)**

| | Control group (suspension) | Microsphere injection |
|---|---|---|
| Two days after administration | 0.54 ± 0.35 | 0.70 ± 0.26 |
| Seven days after | 0.96 ± 0.54 | 0.18 |
| 14 days after | ≦ Detection limit | 0.17 ± 0.06 |
| 21 days after | ≦ Detection limit | 0.10 ± 0.02 |
| 28 days after | ≦ Detection limit | 0.09 ± 0.02 |

(In Table 1, the betamethasone concentrations in the retina-choroids represent the average of three or four eyes ± standard error. The value after seven days of the microsphere injection represents the average of two eyes since the concentrations were the detection limit or lower in two eyes of four eyes.)

### 4. Choroidal neovascularization inhibition tests

Inhibitory effects of the betamethasone-containing microsphere injection on choroidal neovascularization were studied by the method below using laser-induced rat choroidal neovascularization models. As a control, using a microsphere injection containing only the solvent (an aqueous solution containing 0.4% (w/v) of polysorbate 80 and 2.6% (w/v) of glycerin), operation was carried out in the same manner.
1) A one ml/kg mixed solution (7:1) of a 5% (w/v) ketamine hydrochloride injection and a 2% (w/v) xylazine hydrochloride injection was administered intramuscularly to rats to anesthetize them systemically. A 0.5% (w/v) tropicamide/0.5% (w/v) phenylephrine hydrochloride ophthalmic solution was instilled into the eyes to cause mydriasis, and then photocoagulation was performed with a krypton laser photocoagulation apparatus. The photocoagulation was carried out in a posterior section of ocular fundus at eight spots per eye sparsely avoiding thick retinal vessels and focusing on the retinal depth (coagulation conditions: spot size: 100 µm, output: 100 mW, coagulation time: 0.1 sec). After the photocoagulation, the ocular fundus was photographed to confirm laser irradiation sites.
2) Immediately after the photocoagulation, the betamethasone-containing microsphere infection was subconjunctivally administered to an upper portion of each rat in an amount of 50 µl per eye with a micro syringe equipped with a 30 G needle. The microsphere injection containing only the solvent (an aqueous solution containing 0.4% (w/v) of polysorbate 80 and 2.6% (w/v) of glycerin) was subconjunctivally administered to an upper portion of the control group in an amount of 50 µ l per eye.
3) Fourteen and 28 days after the photocoagulation, 0.1 ml of a 10% (w/v) aqueous fluorescein solution was injected from a tail vein, and fluorescein angiography was performed. In the fluorescein angiography, a spot where fluorescence diapedesis was not observed was judged as negative, and a spot where fluorescence diapedesis was observed was judged as positive. Each neovascularization exhibition rate (%) was calculated from a rate of a positive spot number to eight spots irradiated with the laser according to the following calculation equation. With regard to spots which exhibit slightly excessive fluorescence, forming of two spots was judged as positive of one count.

Neovascularization exhibition rate (%) = (fluorescence diapedesis spot number / laser irradiation spot number) × 100 The obtained results are expressed in the average ± standard error. A Student's t test was used for statistical analysis. Each level of significance was taken as 5% on both sides.

Inhibitory effects of the betamethasone-containing microsphere on the choroidal neovascularization are shown in Table 2. While a neovascularization exhibition rate of the control group 14 days after the photocoagulation was 60.9 ± 4.4%, a neovascularization exhibition rate of a betamethasone-containing microsphere group was 12.5 ± 2.4%, and the betamethasone-containing microsphere exhibited a statistically significant inhibitory action on the choroidal neovascularization. Even 28 days after the photocoagulation, while a neovascularization exhibition rate of the control group was 73.4 ± 6.0%, a neovascularization exhibition rate of the betamethasone-containing microsphere group was 12.5 ± 2.4%, and the betamethasone-containing microsphere exhibited the statistically significant inhibitory action on the choroidal neovascularization. The above-mentioned results mean that the subconjunctivally administered betamethasone-containing microsphere exhibits the inhibitory action on the choroidal neovascularization even 14 and 28 days after the administration.

**Table 2: Neovascularization exhibition rates (%) of betamethasone-containing microsphere**

| | Control group | Microsphere group |
|---|---|---|
| After 14 days | 60.9 ± 4.4 | 12.5 ± 2.4 |
| After 28 days | 73.4 ± 6.0 | 12.5 ± 2.4 |

(In Table 2, the neovascularization exhibition rates of the respective groups represent the average of eight eyes ± standard error.)

### Industrial Applicability

The present invention can provide an excellent drug delivery system to posterior segments by subconjunctival administration.

## Claims

1. A drug delivery system **characterized in that** it comprises an injection which comprises fine particles with a particle diameter of 50 nm to 150 µm, which are made of a biodegradable or biosoluble polymer and which contain a drug, for use in treating or preventing a disease of a posterior segment, wherein said drug delivery system is subconjunctivally administered.

2. A subconjunctival injection which comprises fine particles with a particle diameter of 50 nm to 150 µm, which are made of a biodegradable or biosoluble polymer and which contain a drug, for use in treating or preventing a disease of a posterior segment, wherein said subconjunctival injection enables the drug to deliver to a posterior segment.

3. The drug delivery system as claimed in claim 1 and the subconjunctival injection as claimed in claim 2, wherein the posterior segment is a retina, a choroid, an optic nerve, a vitreous body or a crystalline lens.

4. The drug delivery system as claimed in claim 1 and the subconjunctival injection as claimed in claim 2, wherein the drug is a drug for treatment or prevention of a disease of a retina, a choroid, an optic nerve, a vitreous body or crystalline lens.

5. The drug delivery system as claimed in claim 1 and the subconjunctival injection as claimed in claim 2, wherein the drug is an anti-inflammatory, an immunosuppressor, an antiviral, an anticancer drug, an angiogenesis inhibitor, an antithrombotic agent, an optic neural protectant, an antimicrovial or an antifungal agent.

6. The drug delivery system as claimed in claim 1 and the subconjunctival injection as claimed in claim 2, wherein the drug is a steroid or derivative thereof.

7. The drug delivery system as claimed in claim 1 and the subconjunctival injection as claimed in claim 2, wherein the particle diameter of the fine particles is 50 nm to 75 µm.

8. The drug delivery system as claimed in claim 1 and the subconjunctival injection as claimed in claim 2, wherein the fine particles are made of poly(lactic acid) or lactic acid-glycolic acid copolymers.

9. The drug delivery system as claimed in claim 1 and the subconjunctival injection as claimed in claim 2, wherein the drug is a steroid or derivative thereof, the particle diameter of the fine particles is 50 nm to 75 µm, and the fine particles are made of poly(lactic acid) or lactic acid-glycolic acid copolymers.

## Patentansprüche

1. Arzneimittelabgabesystem, **dadurch gekennzeichnet, dass** es eine Injektion umfasst, welche feine Teilchen mit einem Teilchendurchmesser von 50 nm bis 150 µm umfasst, welche aus einem bioabbaubaren oder biolöslichen Polymer hergestellt sind und welche ein Arzneimittel enthalten, zur Verwendung bei der Behandlung oder Verhinderung einer Krankheit eines posterioren Segments, wobei das Arzneimittelabgabesystem subkonjunktival verabreicht wird.

2. Subkonjunktivale Injektion, welche feine Teilchen mit einem Teilchendurchmesser von 50 nm bis 150 µm umfasst, welche aus einem bioabbaubaren oder biolöslichen Polymer hergestellt sind und welche ein Arzneimittel enthalten, zur Verwendung bei der Behandlung oder Verhinderung einer Krankheit eines posterioren Segments, wobei die subkonjunktivale Injektion es ermöglicht, das Arzneimittel an ein posteriores Segment abzugeben.

3. Arzneimittelabgabesystem nach Anspruch 1 und subkonjunktivale Injektion nach Anspruch 2, wobei das posteriore Segment eine Retina, eine Chorioidea, ein Sehnerv, ein glasartiger Körper oder eine kristalline Linse ist.

4. Arzneimittelabgabesystem nach Anspruch 1 und subkonjunktivale Injektion nach Anspruch 2, wobei das Arzneimittel ein Arzneimittel zur Behandlung oder Verhinderung einer Krankheit einer Retina, einer Chorioidea, eines Sehnervs, eines glasartigen Körpers oder einer kristallinen Linse ist.

5. Arzneimittelabgabesystem nach Anspruch 1 und subkonjunktivale Injektion nach Anspruch 2, wobei das Arzneimittel ein Entzündungshemmer, ein Immunosuppressor, ein Antivirusmittel, ein Antikrebs-Arzneimittel, ein Angiogenese-Inhibitor, ein antithrombotisches Mittel, ein sehneurales Schutzmittel, ein antimikroviales oder ein antimykotisches Mittel ist.

6. Arzneimittelabgabesystem nach Anspruch 1 und subkonjunktivale Injektion nach Anspruch 2, wobei das Arzneimittel ein Steroid oder Derivat hiervon ist.

7. Arzneimittelabgabesystem nach Anspruch 1 und subkonjunktivale Injektion nach Anspruch 2, wobei der Teilchendurchmesser der feinen Teilchen 50 nm bis 75 µm beträgt.

8. Arzneimittelabgabesystem nach Anspruch 1 und subkonjunktivale Injektion nach Anspruch 2, wobei die feinen Teilchen aus Polymilchsäure oder Milchsäure-Glykolsäure-Copoloymeren hergestellt sind.

9. Arzneimittelabgabesystem nach Anspruch 1 und subkonjunktivale Injektion nach Anspruch 2, wobei das Arzneimittel ein Steroid oder Derivat hiervon ist, der Teilchendurchmesser der feinen Teilchen 50 nm bis 75 µm beträgt, und die feinen Teilchen aus Polymilchsäure oder Milchsäure-Glykolsäure-Copolymeren hergestellt sind.

## Revendications

1. Système de délivrance de médicament **caractérisé en ce qu'**il comprend une composition injectable qui comprend de fines particules ayant une granulométrie de 50 nm à 150 µm, qui sont faites d'un polymère biodégradable ou biosoluble et qui contiennent un médicament, pour utilisation dans le traitement ou la prévention d'une maladie d'un segment postérieur, lequel système de délivrance de médicament est administré par voie sous-conjonctivale.

2. Composition injectable par voie sous-conjonctivale qui comprend de fines particules ayant une granulométrie de 50 nm à 150 µm, qui sont faites d'un polymère biodégradable ou biosoluble et qui contiennent un médicament, pour utilisation dans le traitement ou la prévention d'une maladie d'un segment postérieur, laquelle composition injectable par voie sous-conjonctivale permet au médicament d'être délivré à un segment postérieur.

3. Système de délivrance de médicament selon la revendication 1 et composition injectable par voie sous-conjonctivale selon la revendication 2, dans lesquels le segment postérieur est une rétine, une choroïde, un nerf optique, un corps vitré ou un cristallin.

4. Système de délivrance de médicament selon la revendication 1 et composition injectable par voie sous-conjonctivale selon la revendication 2, dans lesquels le médicament est un médicament pour traiter ou prévenir une maladie de la rétine, de la choroïde, du nerf optique, du corps vitré ou du cristallin.

5. Système de délivrance de médicament selon la revendication 1 et composition injectable par voie sous-conjonctivale selon la revendication 2, dans lesquels le médicament est un anti-inflammatoire, un immunodépresseur, un antiviral, un médicament anticancéreux, un inhibiteur de l'angiogenèse, un agent antithrombotique, un protecteur des nerfs optiques, un antimicrobien ou un agent antifongique.

6. Système de délivrance de médicament selon la revendication 1 et composition injectable par voie sous-conjonctivale selon la revendication 2, dans lesquels le médicament est un stéroïde ou un dérivé de celui-ci.

7. Système de délivrance de médicament selon la revendication 1 et composition injectable par voie sous-conjonctivale selon la revendication 2, dans lesquels la granulométrie des fines particules est de 50 nm à 75 µm.

8. Système de délivrance de médicament selon la revendication 1 et composition injectable par voie sous-conjonctivale selon la revendication 2, dans lesquels les fines particules sont faites en poly(acide lactique) ou en copolymère d'acide lactique/acide glycolique.

9. Système de délivrance de médicament selon la revendication 1 et composition injectable par voie sous-conjonctivale selon la revendication 2, dans lesquels le médicament est un stéroïde ou un dérivé de celui-ci, la granulométrie des fines particules est de 50 nm à 75 µm, et les fines particules sont faites en poly(acide lactique) ou en copolymère d'acide lactique/acide glycolique.
